# FASCICULE DE BREVET EUROPEEN

(11) **EP 0 908 114 B1**
(45) Date de publication et mention de la délivrance du brevet: **19.12.2001**
(21) Numéro de dépôt: 98402284.8
(22) Date de dépôt: 16.09.1998
(51) Int. Cl.: A45D 34/04, A61M 35/00

(54) **Ensemble de conditionnement et d'application notamment d'un produit cosmétique**
Verpackungseinheit zum Aufbewahren und Auftragen eines Produktes, insbesondere eines kosmetischen Produktes
Device for storing and applying a product, in particular a cosmetic product

(30) Priorité: 10.10.1997 FR 9712699
(43) Date de publication de la demande: 14.04.1999
(73) Titulaire: L'OREAL, 75008 Paris (FR)
(72) Inventeur: Vieu, Valérie, 75016 Paris (FR)
(74) Mandataire: Boulard, Denis

(56) Documents cités:
- DE-A- 3 318 696
- FR-A- 843 463
- US-A- 2 002 144
- US-A- 2 096 858
- US-A- 2 511 557
- US-A- 3 226 754
- US-A- 4 177 811

## Description

La présente invention a trait à un ensemble de conditionnement et d'application d'un produit cosmétique.

Traditionnellement, les crèmes telles que les crèmes de soin, teintées ou non, les gels, sont conditionnées dans des pots à large ouverture. Le prélèvement du produit en vue de son application, notamment sur le visage se fait avec les doigts. Un des problèmes liés au prélèvement manuel d'une crème dans un pot, tient au fait que ce mode de prélèvement est source de contamination microbienne pour le contenu du pot. Dans le cas d'une crème ou d'un gel teinté, s'ajoute au problème d'hygiène le fait qu'il faut se laver les mains après application.

Le brevet FR 2 432 287 décrit un applicateur de type doigtier dont une extrémité est solidaire d'un applicateur de type tampon de mousse. Le doigtier décrit dans ce document se présente sous forme de deux demi coquilles articulées l'une sur l'autre au moyen d'une charnière, permettant le serrage du doigt de l'utilisatrice à l'intérieur des deux coquilles. Au bout du doigtier est fixé un applicateur sous forme d'un tampon de mousse. Outre la complexité du dispositif, celui-ci n'est pas adapté à l'application de crèmes, notamment de crèmes de soin, nécessitant un massage suffisant pour faire pénétrer le produit dans la peau. Par ailleurs, l'applicateur étant situé à distance du doigt, l'utilisatrice n'a pas les sensations tactiles que procurent l'application manuelle. Enfin, la précision à l'application s'en trouve réduite.

Le brevet US 4 998 838 décrit un tube ou récipient déformable, surmonté d'un tampon applicateur en matière poreuse, un applicateur auxiliaire étant contenu de manière amovible à l'intérieur d'un capuchon, apte à recouvrir le tampon surmontant le tube. Pour appliquer du produit sur le visage, l'utilisatrice presse les parois du tube de manière à provoquer la sortie du produit sur le tampon applicateur. Le produit présent sur l'applicateur est déposé sur la surface à traiter, puis est étalé au moyen de l'applicateur auxiliaire. Une telle conception est relativement coûteuse en ce qu'elle dissocie la fonction prélèvement et application du produit, de la fonction étalement. En outre, de la même manière que pour le brevet mentionné précédemment, l'utilisatrice n'a pas les sensations tactiles que procure l'application manuelle. Enfin, un tel concept serait difficilement adaptable sur un pot tel qu'utilisé conventionnellement pour le conditionnement des gels ou crèmes de soin.

Aussi, est-ce un des objets de l'invention que de fournir un ensemble de conditionnement et d'application résolvant en tout ou partie les problèmes discutés en référence aux dispositifs de la technique antérieure.

C'est en particulier un objet de l'invention que de pouvoir permettre un mode d'application d'un produit, permettant de conserver de manière relativement stérile, le produit à appliquer, tout en conservant à l'application, la précision et les sensations tactiles que procure l'application manuelle d'un tel produit.

C'est un autre objet de l'invention que de fournir un ensemble de conditionnement et d'application, n'augmentant pas le volume des conditionnements conventionnels, et comportant un organe d'application toujours prêt à l'application.

D'autres objets apparaîtront de manière détaillée dans la description qui suit.

Selon l'invention, ces objets sont atteints en réalisant un ensemble de conditionnement et d'application d'un produit cosmétique comprenant : un récipient d'axe X dont une extrémité délimite une ouverture ; ledit récipient contenant un produit cosmétique; des moyens pour obturer de manière amovible ladite ouverture ; un organe d'application sous forme d'un doigt de gant, en matériau souple et fin, dont une extrémité ouverte est solidaire d'un élément annulaire rigide ou semi rigide, ledit doigt de gant étant apte à recevoir l'un des doigts d'une utilisatrice pour prélever du produit à l'intérieur du récipient ; des moyens pour, dans une position de stockage, permettre un positionnement dudit élément annulaire au voisinage de ladite ouverture, lorsque ledit organe d'application est disposé à l'intérieur du récipient ; et des moyens de rigidification s'étendant sur au moins une partie de la hauteur axiale de l'organe d'application pour, en position de stockage, lors du retrait du doigt de l'utilisatrice, hors dudit doigt de gant, limiter le retournement de ce dernier ; ledit organe d'application lorsque il est disposé à l'intérieur du récipient étant en contact avec ledit produit.

On réalise ainsi un ensemble de conditionnement et d'application, dans lequel l'application est réalisée par le doigt, via la fine membrane constituant le doigt de gant. La précision à l'application, ainsi que les sensations tactiles sont quasi intactes. Le doigt n'est jamais en contact avec le produit, ce qui protège ce dernier de toute contamination, notamment microbienne. Certes, le doigt de gant est amené au contact de la peau, notamment du visage, puis du produit à l'intérieur du récipient. Toutefois, les risques de contamination sont sensiblement plus faibles que celles résultant du contact du produit avec les doigts. Lorsque, après application, le doigt de gant est remis en position à l'intérieur du récipient, il est prêt pour l'utilisation suivante sans avoir à faire de manipulations préalables. L'encombrement de l'ensemble n'est pas augmenté de manière sensible. En outre la protection du produit peut encore être améliorée en imprégnant le doigt de gant d'une composition anti-microbienne.

Avantageusement, le doigt de gant est dimensionné de manière à permettre le prélèvement de sensiblement tout le contenu du récipient. Un tel dimensionnement, notamment dans le cas d'un doigt de gant en latex, peut résulter d'un allongement par élasticité du doigt de gant.

Le doigt de gant peut être rendu solidaire dudit élément annulaire par soudure, collage, ou accrochage mécanique, notamment par claquage à l'intérieur d'une gorge annulaire formée par ledit élément annulaire.

Selon un mode de réalisation, le doigt de gant et l'élément annulaire sont obtenus par bi-injection de deux matériaux physico-chimiquement compatibles. Le même matériau peut être utilisé pour les deux, en jouant seulement sur les épaisseurs.

Avantageusement, les moyens de rigidification sont solidaires de l'élément annulaire.

Avantageusement encore, les moyens de rigidification sont constitués d'un élément situé en regard de tout ou partie de la périphérie du doigt de gant, et s'étendant depuis l'élément annulaire, sur une partie de la hauteur axiale de l'organe d'application. Les moyens de rigidification peuvent être situés à l'intérieur du doigt de gant. Toutefois, pour des raisons de confort à l'utilisation, il est préférable que les moyens de rigidification soient situés à l'extérieur du doigt de gant.

Selon un premier mode de réalisation, les moyens de rigidification sont constitués d'un manchon cylindrique. A titre d'indication, ledit manchon s'étend sur environ ¼ à 1/3 de la hauteur axiale de l'organe d'application. Il est évident que plus le manchon est long, et moins le doigt est souple, ce qui peut être pénalisant lors de l'application.

Dans cette hypothèse, l'élément annulaire, les moyens de rigidification, et le doigt de gant peuvent former une seule pièce obtenue de moulage d'un matériau thermoplastique, le doigt de gant présentant une zone de plus grande épaisseur s'étendant depuis l'élément annulaire, de manière à former les moyens de rigidification, et une zone de plus faible épaisseur s'étendant depuis la zone de plus grande épaisseur jusqu'à un fond fermé formé par ledit doigt de gant. On peut utiliser notamment un polyéthylène basse densité.

Selon un autre mode de réalisation, les moyens de rigidification sont constitués d'au moins deux pattes orientées axialement. A titre d'exemple, on utilise deux pattes de rigidification s'étendant sur une partie substantielle de la longueur de l'organe d'application. En position d'utilisation, les deux pattes sont situées de chaque côté du doigt, à la manière d'attelles. Des moyens de repérage peuvent être prévus pour permettre un bon positionnement du doigt à l'intérieur du doigt de gant. Dans ce mode de réalisation, le retournement du doigt de gant, lors du retrait du doigt est très faible, voire inexistant.

Selon un autre mode de réalisation, lesdites pattes s'étendent sur environ ¼ à ¾ de la hauteur axiale de l'organe d'application. Dans cette hypothèse, on peut utiliser trois ou quatre pattes disposées régulièrement tout autour du doigt de gant.

Les moyens pour, dans une position de stockage, permettre un positionnement dudit élément annulaire au voisinage de ladite ouverture, lorsque ledit organe d'application est disposé à l'intérieur du récipient, peuvent comporter une gorge annulaire ménagée dans une collerette transversale formée par le bord libre d'un col surmontant le récipient, et apte à recevoir ledit élément annulaire. Dans ce cas de figure, lors du retrait du doigt, l'utilisatrice retient de l'autre main, l'élément annulaire sur le col du récipient.

Les moyens d'obturation peuvent comporter un bouchon présentant une jupe interne apte, en position fermée des moyens d'obturation à venir appuyer sur ledit élément annulaire, de manière à le maintenir dans ladite gorge. Un tel bouchon peut être vissé sur le col du récipient.

A titre d'exemples non limitatifs, le doigt de gant est réalisé en un matériau tels que les polyéthylènes basse densité, les latex, les élastomères thermoplastiques, les non tissés de polyéthylène ou de polypropylène, etc.. Dans la pratique, peut être utilisé tout matériau souple, étanche au produit contenu dans le récipient, et compatible avec le produit de manière à ne pas se dégrader au contact du produit et à ne pas affecter les propriétés du produit.

Le produit est prélevé, notamment au voisinage d'une extrémité fermée du doigt de gant, opposée à l'extrémité ouverte, le doigt de gant pouvant, au moins au voisinage de ladite extrémité fermée, présenter une épaisseur comprise entre 0,1 mm et 1 mm.

Le produit peut être un produit cosmétique, notamment une crème de soin, un gel, ou un fond de teint.

L'invention consiste, mises à part les dispositions exposées ci-dessus, en un certain nombre d'autres dispositions qui seront explicitées ci-après, à propos d'exemples de réalisation non limitatifs, décrits en référence aux figures annexées, parmi lesquelles :
- la figure 1 représente de façon schématique un mode de réalisation préféré de l'ensemble de conditionnement et d'application selon l'invention;
- les figures 2A-2C illustrent partiellement différents modes de réalisation d'un applicateur utilisé dans l'ensemble de conditionnement et d'application selon l'invention, et
- les figures 3A-3C illustrent différents modes de réalisation de l'organe de rigidification tel que pouvant être utilisé dans un applicateur selon l'invention.

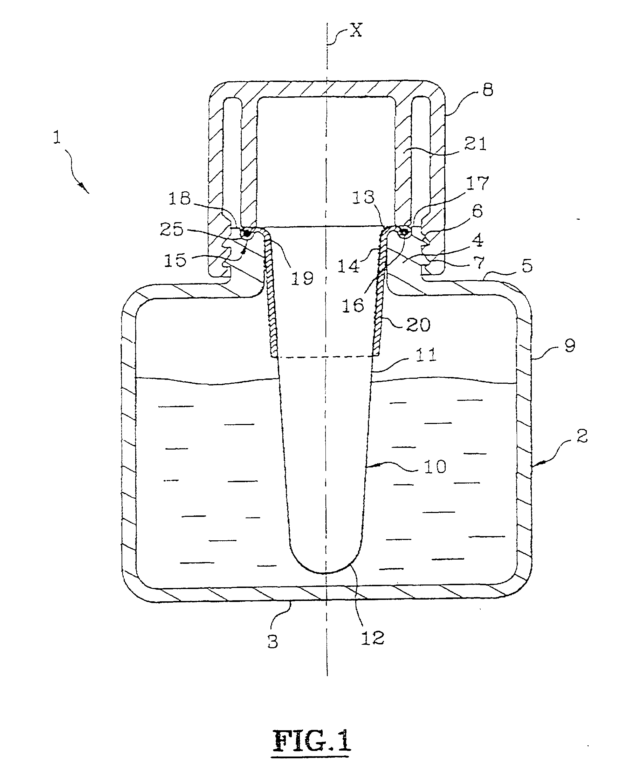

L'ensemble de conditionnement et d'application 1 représenté à la figure 1 comporte un flacon 2 d'axe X, notamment en verre ou en matériau thermoplastique (polypropylène). Le flacon 2 présente un corps 9 dont une extrémité est fermée par un fond 3. L'autre extrémité est surmontée d'un col 4 relié au corps 3 par un épaulement 5. Le col 4 présente un bord libre 18 délimitant une ouverture 19. La surface extérieure du col comporte un filetage 6 apte à coopérer avec un filetage correspondant 7 prévu sur la surface interne d'un bouchon 8.

A l'intérieur du récipient 2 est disposé l'organe d'application 10, lequel présente un doigt de gant 11, en latex par exemple, dont une extrémité 12 est fermée, et se situe sensiblement au voisinage du fond 3 du flacon. L'autre extrémité 14 du doigt de gant délimite une ouverture 13 rendue solidaire d'un organe annulaire 15 rigide ou semi rigide, formant un bourrelet 25 qui, en position montée est disposé à l'intérieur d'une gorge 16 réalisée dans une collerette transversale 17 formée par le bord libre 18 du col 4. L'organe annulaire 15 se prolonge à l'intérieur du récipient par un manchon 20 disposé tout autour du doigt de gant 11, et s'étendant sur environ un tiers de la hauteur axiale du doigt de gant 11. Le manchon 20 est de section telle qu'en position montée illustrée à la figure 1, il obture de façon quasi-étanche l'ouverture 19. Le montage peut être légèrement auto-serrant de manière à favoriser la tenue de l'applicateur 10 dans l'ouverture 19, et lors du retrait de l'applicateur du flacon, l'essorage de ladite partie rigide. Des stries ou rainures peuvent être prévues sur la surface interne du col 4 du récipient, ou sur la surface extérieure du manchon, de manière à éviter le pistonnage lors du retrait de l'applicateur, et à évacuer l'air lors de l'insertion de l'applicateur dans le col du récipient 2. Sur sa partie située en regard du doigt de gant, la surface interne du manchon 20 peut être collée sur la surface extérieure du doigt de gant de manière à éviter le retournement du doigt de gant lorsque l'utilisatrice, après application, retire son doigt. Ceci sera discuté plus en détail par la suite.

Le bouchon 8 comporte une jupe interne 21 qui, en position fermée du bouchon appuie sur l'élément annulaire 15, de manière à améliorer le maintien de l'applicateur 10 dans l'ouverture 19.

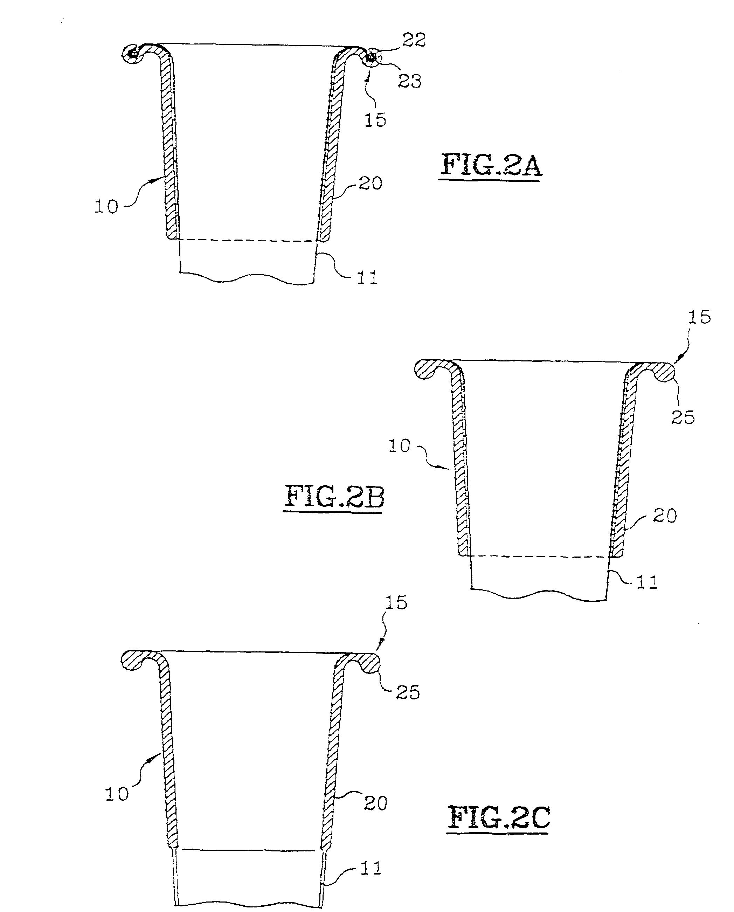

Les figures 2A-2C auxquelles il est maintenant fait référence illustrent différents modes de réalisation de l'applicateur 10. A la figure 2A, l'extrémité ouverte du doigt de gant en matériau souple, forme un bourrelet torique 22, disposé par claquage dans une gorge 23 formée par l'élément annulaire 15. Le doigt de gant 11 est collé sur la surface intérieure du manchon 20 prolongeant ledit élément annulaire 15.

Dans le mode de réalisation de la figure 2B, l'organe annulaire 15, le manchon, 20, et le doigt de gant 11 sont formés par bi-injection de deux matériaux compatibles physico-chimiquement. Ainsi, on réalise l'élément annulaire 15 et le manchon 20 en polypropylène. On surinjecte ensuite le doigt de gant 11 en polyéthylène. Les deux matériaux utilisés étant compatibles, le collage du manchon sur la surface extérieure du doigt de gant se fait de manière automatique par fusion des matériaux.

Dans le mode de réalisation, de la figure 2C, l'organe annulaire 15, le manchon de rigidification 20, et le doigt de gant 11 sont réalisés de moulage d'une seule pièce, en utilisant un matériau tel qu'un polyéthylène basse densité. Lors du moulage, on joue seulement sur les épaisseurs du matériau pour réaliser d'une part le manchon de rigidification 20 (de plus grande épaisseur) et d'autre part, la partie d'application et de prélèvement du doigt de gant 11 (de plus faible épaisseur), située sous le manchon 20.

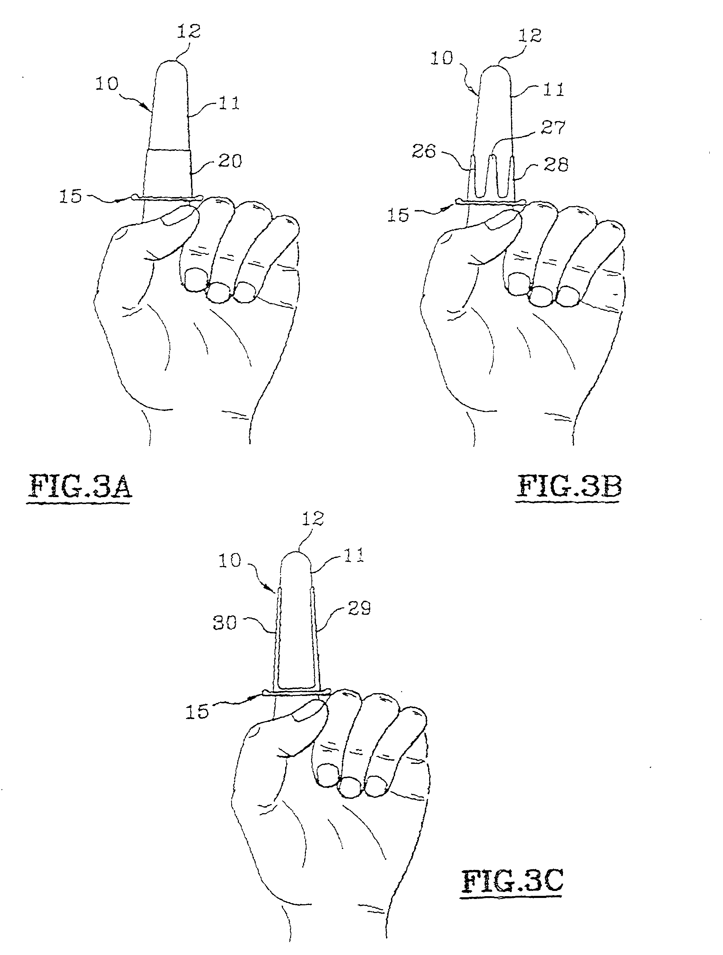

Les figures 3A-3C illustrent différentes formes pour l'organe permettant de limiter, lors du retrait du doigt de l'utilisatrice, le retournement du doigt de gant, lequel retournement, s'il était complet, rendrait ensuite difficile toute utilisation ultérieure de l'applicateur 10. Dans le mode de réalisation de la figure 3A, les moyens de rigidification se présentent sous forme d'un manchon rigide 20 s'étendant sur environ 1/3 de la hauteur axiale du doigt de gant 11.

Dans le mode de réalisation de la figure 3B, l'organe de rigidification forme une couronne présentant une pluralité de pattes 26, 27, 28, espacées régulièrement tout autour du doigt de gant, et orientées sensiblement selon l'axe du doigt de gant. L'extrémité libre des pattes est dirigée vers le fond 12 du doigt de gant. De la même manière que pour le mode de réalisation précédent, les pattes s'étendent sur environ 1/3 de la hauteur axiale du doigt de gant 11.

Dans le mode de réalisation de la figure 3C, l'organe de rigidification forme deux pattes latérales 29, 30 disposées, à la manière d'attelles, de part et d'autre du doigt, et s'étendant sur environ ¾ de la hauteur axiale du doigt de gant 11. Des moyens de repérage peuvent être prévus pour, lors de l'insertion du doigt de l'utilisatrice dans le doigt de gant 11, permettre un bon positionnement angulaire du doigt par rapport aux pattes 29, 30.

Pour utiliser l'ensemble selon l'invention, l'utilisatrice, après avoir enlevé le bouchon 8, insère son doigt (par exemple l'index) dans le doigt de gant 11 au travers de l'ouverture 13 qu'il définit. Elle déplace alors éventuellement son doigt dans le flacon de manière à permettre un prélèvement suffisant de produit. Elle retire alors le doigt du flacon, habillé, comme illustré aux figures 3A-3C, de l'applicateur 10, chargé de produit. Eventuellement, dans le cas d'un montage auto-serrant de l'applicateur dans le col 4 du récipient, l'extraction du doigt est favorisée en tournant légèrement le doigt. Ce mouvement permet en outre un essorage de la partie rigide de l'applicateur. Elle applique ensuite le produit sur la surface à traiter, et l'étale de manière appropriée, au moyen du doigt portant le doigt de gant. Après application, elle remet l'applicateur dans le col 4 du flacon. De la main autre que celle portant l'applicateur 10, elle retient l'élément annulaire 15 de manière à le maintenir sur la collerette 17 formée par le col du flacon, et simultanément, retire son doigt. En fonction de la longueur des moyens de rigidification, il se produit un certain retournement d'une partie du doigt de gant, à savoir sur la hauteur du doigt de gant non située en regard des moyens de rigidification. Toutefois, ce retournement n'est pas apte à gêner de manière sensible pour une utilisation ultérieure de l'applicateur. Elle referme ensuite le flacon au moyen du bouchon 8. L'ensemble est ainsi prêt pour une nouvelle utilisation. Le doigt servant à l'application est resté propre, et le produit exempt de toute contamination sensible apte à en affecter les propriétés.

Dans la description détaillée qui précède, il a été fait référence à des modes de réalisation préférés de l'invention. Il est évident que des variantes peuvent y être apportées sans s'écarter de l'invention telle que revendiquée ci-après.

## Revendications

1. Ensemble de conditionnement et d'application (1) d'un produit cosmétique comprenant: un récipient (2) d'axe X, dont une extrémité présente une ouverture (19) ; ledit récipient contenant un produit cosmétique; des moyens (8) pour obturer de manière amovible ladite ouverture (19) ; un organe d'application (10) sous forme d'un doigt de gant (11), en matériau souple et fin, dont une extrémité ouverte (14) est solidaire d'un élément annulaire (15) rigide ou semi rigide, ledit doigt de gant (11) étant apte à recevoir l'un des doigts d'une utilisatrice pour prélever du produit à l'intérieur du récipient ; des moyens (16) pour, dans une position de stockage, permettre un positionnement dudit élément annulaire (15) au voisinage de ladite ouverture (19), lorsque ledit organe d'application (10) est disposé à l'intérieur du récipient (2); et des moyens de rigidification (20, 26-30) s'étendant sur au moins une partie de la hauteur axiale de l'organe d'application (10) pour, en position de stockage, lors du retrait du doigt de l'utilisatrice, hors dudit doigt de gant (11), limiter le retournement de ce dernier ; ledit organe d'application lorsque il est disposé à l'intérieur du récipient étant en contact avec ledit produit.

2. Ensemble de conditionnement et d'application selon la revendication 1 **caractérisé en ce que** le doigt de gant (11) est dimensionné de manière à permettre le prélèvement de sensiblement tout le contenu du récipient (2).

3. Ensemble de conditionnement et d'application selon la revendication 1 ou 2 **caractérisé en ce que** le doigt de gant (11) est solidaire dudit élément annulaire (15) par soudure, collage, ou accrochage mécanique, notamment par claquage à l'intérieur d'une gorge annulaire (23) formée par ledit élément annulaire (15).

4. Ensemble de conditionnement et d'application selon la revendication 1 ou 2 **caractérisé en ce que** le doigt de gant (11) et l'élément annulaire (15) sont obtenus par bi-injection de deux matériaux physico-chimiquement compatibles.

5. Ensemble de conditionnement et d'application selon la revendication 1 **caractérisé en ce que** les moyens de rigidification (20, 26-30) sont solidaires de l'élément annulaire (15).

6. Ensemble de conditionnement et d'application selon la revendication 5 **caractérisé en ce que** les moyens de rigidification (20, 26-30) sont constitués d'un élément situé en regard de tout ou partie de la périphérie du doigt de gant (11), et s'étendant depuis l'élément annulaire (15), sur une partie de la hauteur axiale de l'organe d'application (10).

7. Ensemble de conditionnement et d'application selon la revendication 6 **caractérisé en ce que** les moyens de rigidification sont constitués d'un manchon cylindrique (20).

8. Ensemble de conditionnement et d'application selon la revendication 7 **caractérisé en ce que** ledit manchon (20) s'étend sur environ ¼ à 1/3 de la hauteur axiale de l'organe d'application (10).

9. Ensemble de conditionnement et d'application selon la revendication 7 ou 8 **caractérisé en ce que** l'élément annulaire (15), les moyens de rigidification (20, 26-30), et le doigt de gant (11) forment une seule pièce obtenue de moulage d'un matériau thermoplastique, le doigt de gant (11) présentant une zone de plus grande épaisseur s'étendant depuis l'élément annulaire (15), de manière à former les moyens de rigidification (20), et une zone de plus faible épaisseur s'étendant depuis la zone de plus grande épaisseur jusqu'à un fond fermé (12) formé par ledit doigt de gant (11).

10. Ensemble de conditionnement et d'application selon la revendication 5 ou 6 **caractérisé en ce que** les moyens de rigidification sont constitués d'au moins deux pattes (29, 30) orientées selon l'axe X.

11. Ensemble de conditionnement et d'application selon la revendication 10 **caractérisé en ce que** lesdites pattes (29, 30) s'étendent sur environ ¼ à ¾ de la hauteur axiale de l'organe d'application (10).

12. Ensemble de conditionnement et d'application selon l'une quelconque des revendications 1 à 11 **caractérisé en ce que** lesdits moyens de rigidification (20, 26-30) sont situés à l'extérieur du doigt de gant (11).

13. Ensemble de conditionnement et d'application selon l'une quelconque des revendications précédentes **caractérisé en ce que** les moyens pour, dans une position de stockage, permettre un positionnement dudit élément annulaire (15) au voisinage de ladite ouverture (19), lorsque ledit organe d'application est disposé à l'intérieur du récipient, comportent une gorge annulaire (16) ménagée dans une collerette transversale (17) formée par un bord libre (18) d'un col (4) surmontant le récipient, et apte à recevoir ledit élément annulaire (15).

14. Ensemble de conditionnement et d'application selon la revendication 13 **caractérisé en ce que** les moyens d'obturation (8) comportent un bouchon présentant une jupe interne (21) apte, en position fermée des moyens d'obturation (8) à venir appuyer sur ledit élément annulaire (15), de manière à le maintenir dans ladite gorge (16).

15. Ensemble de conditionnement et d'application selon la revendication 14 **caractérisé en ce que** le bouchon (8) est vissé sur le col (4) du récipient.

16. Ensemble de conditionnement et d'application selon l'une quelconque des revendications précédentes **caractérisé en ce que** le doigt de gant (11) est réalisé en un matériau tels que les polyéthylènes basse densité, les latex, les élastomères thermoplastiques, les non tissés de polyéthylène ou de polypropylène, etc..

17. Ensemble de conditionnement et d'application selon l'une quelconque des revendications précédentes **caractérisé en ce que** le produit est prélevé au voisinage d'une extrémité fermée (12) du doigt de gant (11), opposée à l'extrémité ouverte (14), le doigt de gant étant, au moins au voisinage de ladite extrémité fermée (12) d'épaisseur comprise entre 0,1 mm et 1 mm.

18. Utilisation d'un ensemble de conditionnement et d'application selon l'une quelconque des revendications précédentes pour un produit cosmétique, notamment une crème de soin, un gel, ou un fond de teint.

## Patentansprüche

1. Verpackungs- und Auftrageinheit (1) für ein kosmetisches Produkt, die aufweist: einen Behälter (2) mit einer Achse X, von dem ein Ende eine Öffnung (19) aufweist; wobei der Behälter ein kosmetisches Produkt enthält; Mittel (8), um die Öffnung (19) lösbar zu verschließen; ein Auftragorgan (10) in Form eines Handschuhfingers (11) aus geschmeidigem und dünnem Material, von dem ein offenes Ende (14) fest mit einem steifen oder halbsteifen, ringförmigen Element (15) verbunden ist, wobei der Handschuhfinger (11) einen der Finger einer Benutzerin aufnehmen kann, um Produkt innerhalb des Behälters zu entnehmen; Mittel (16), um in einer Lagerstellung eine Positionierung des ringförmigen Elements (15) in der Nähe der Öffnung (19) zu erlauben, wenn das Auftragorgan (10) sich im Inneren des Behälters (2) befindet; und Versteifungsmittel (20, 26-30), die sich über mindestens einen Teil der axialen Höhe des Auftragorgans (10) erstrecken, um in der Lagerposition beim Herausziehen des Fingers der Benutzerin aus dem Handschuhfinger (11) das Umstülpen dieses Handschuhfingers zu begrenzen; wobei das Auftragorgan, wenn es sich innerhalb des Behälters befindet, mit dem Produkt in Kontakt steht.

2. Verpackungs- und Auftrageinheit nach Anspruch 1, **dadurch gekennzeichnet, daß** der Handschuhfinger (11) so bemessen ist, daß er die Entnahme von im wesentlichen dem ganzen Inhalt des Behälters (2) erlaubt.

3. Verpackungs- und Auftrageinheit nach Anspruch 1 oder 2, **dadurch gekennzeichnet, daß** der Handschuhfinger (11) durch Schweißen, Kleben oder mechanische Befestigung, insbesondere durch Einschnappen im Inneren einer vom ringförmigen Element (15) gebildeten Ringkehle (23), fest mit dem ringförmigen Element (15) verbunden wird.

4. Verpackungs- und Auftrageinheit nach Anspruch 1 oder 2, **dadurch gekennzeichnet, daß** der Handschuhfinger (11) und das ringförmige Element (15) durch Bi-Injektion von zwei physikalisch-chemisch kompatiblen Materialien erhalten werden.

5. Verpackungs- und Auftrageinheit nach Anspruch 1, **dadurch gekennzeichnet, daß** die Versteifungsmittel (20, 26-30) fest mit dem ringförmigen Element (15) verbunden sind.

6. Verpackungs- und Auftrageinheit nach Anspruch 5, **dadurch gekennzeichnet, daß** die Versteifungsmittel (20, 26-30) aus einem Element bestehen, das sich vor dem ganzen Umfang oder einem Teil des Umfangs des Handschuhfingers (11) befindet und sich vom ringförmigen Element (15) über einen Teil der axialen Höhe des Auftragorgans (10) erstreckt.

7. Verpackungs- und Auftrageinheit nach Anspruch 6, **dadurch gekennzeichnet, daß** die Versteifungsmittel aus einer zylindrischen Manschette (20) bestehen.

8. Verpackungs- und Auftrageinheit nach Anspruch 7, **dadurch gekennzeichnet, daß** die Manschette (20) sich über etwa 1/4 bis 1/3 der axialen Höhe des Auftragorgans (10) erstreckt.

9. Verpackungs- und Auftrageinheit nach Anspruch 7 oder 8, **dadurch gekennzeichnet, daß** das ringförmige Element (15), die Versteßmgsmittel (20, 26-30) und der Handschuhfinger (11) ein einziges Teil bilden, das in einem Stück aus einem thermoplastischen Material hergestellt wird, wobei der Handschuhfinger (11) eine Zone größerer Dicke, die sich vom ringförmigen Element (15) aus erstreckt, um die Versteifungsmittel (20) zu bilden, und eine Zone geringerer Dicke aufweist, die sich von der Zone größerer Dicke bis zu einem geschlossenen Boden (12) erstreckt, der vom Handschuhfinger (11) gebildet wird.

10. Verpackungs- und Auftrageinheit nach Anspruch 5 oder 6, **dadurch gekennzeichnet, daß** die Versteifungsmittel aus mindestens zwei Laschen (29, 30) bestehen, die gemäß der Achse X ausgerichtet sind.

11. Verpackungs- und Auftrageinheit nach Anspruch 10, **dadurch gekennzeichnet, daß** die Laschen (29, 30) sich über etwa 1/4 bis 3/4 der axialen Höhe des Auftragorgans (10) erstrecken.

12. Verpackungs- und Auftrageinheit nach einem der Ansprüche 1 bis 11, **dadurch gekennzeichnet, daß** die Versteifungsmittel (20, 26-30) sich außerhalb des Handschuhfingers (11) befinden.

13. Verpackungs- und Auftrageinheit nach einem der vorhergehenden Ansprüche, **dadurch gekennzeichnet, daß** die Mittel, um in einer Lagerstellung eine Positionierung des ringförmigen Elements (15) in der Nähe der Öffnung (19) zu ermöglichen, wenn das Auftragorgan sich im Inneren des Behälters befindet, eine Ringkehle (16) aufweisen, die in einem Querkragen (17) ausgebildet ist, der von einem freien Rand (18) eines auf dem Behälter sitzenden Halses (4) gebildet wird und das ringförmige Element (15) aufnehmen kann.

14. Verpackungs- und Auftrageinheit nach Anspruch 13, **dadurch gekennzeichnet, daß** die Verschlußmittel (8) einen Stopfen mit einer inneren Schürze (21) aufweisen, die in der geschlossenen Stellung der Verschlußmittel (8) auf das ringförmige Element (15) drücken kann, um es in der Kehle zu (16) halten.

15. Verpackungs- und Auftrageinheit nach Anspruch 14, **dadurch gekennzeichnet, daß** der Stopfen (8) auf den Hals (4) des Behälters geschraubt wird.

16. Verpackungs- und Auftrageinheit nach einem der vorhergehenden Ansprüche, **dadurch gekennzeichnet, daß** der Handschuhfinger (11) aus einem Material wie zum Beispiel den weichen Polyethylenen, den Latexmaterialien, den thermoplastischen Elastomermaterialien, den Vliesen aus Polyethylen oder Polypropylen, usw. hergestellt wird.

17. Verpackungs- und Auftrageinheit nach einem der vorhergehenden Ansprüche, **dadurch gekennzeichnet, daß** das Produkt in der Nähe eines geschlossenen Endes (12) des Handschuhfingers (11) entgegengesetzt zum offenen Ende (14) entnommen wird, wobei der Handschuhfinger zumindest in der Nähe des geschlossenen Endes (12) eine Dicke zwischen 0,1 mm und 1 mm aufweist.

18. Verwendung einer Verpackungs- und Auftrageinheit nach einem der vorhergehenden Ansprüche für ein kosmetisches Produkt, insbesondere eine Pflegecreme, ein Gel oder einen Fond de Teint.

## Claims

1. Unit (1) for packaging and applying a cosmetic product, comprising: a container (2) of axis X, one end of which has an opening (19); the said container containing a cosmetic product; means (8) for removably plugging the said opening (19); an applicator member (10) in the form of a thimble (11) made of flexible and fine material, an open end (14) of which is secured to a rigid or semirigid annular element (15), the said thimble (11) being capable of accommodating one of the fingers of a user so as to pick up product from inside the container; means (16) for, in a storage position, allowing the said annular element (15) to be positioned near the said opening (19) when the said applicator member (10) is arranged inside the container (2); and rigidifying means (20, 26-30) extending over at least part of the axial height of the applicator member (10) for, in the storage position, as the user withdraws her finger from the said thimble (11), limiting the turninginside-out of the latter; the said applicator member, when arranged inside the container, being in contact with the said product.

2. Packaging and applicator unit according to Claim 1, **characterized in that** the thimble (11) is sized in such a way as to allow practically the entire contents of the container (2) to be picked up.

3. Packaging and applicator unit according to Claim 1 or 2, **characterized in that** the thimble (11) is secured to the said annular element (15) by welding, bonding or mechanical attachment, particularly by snap-fastening inside an annular groove (23) formed by the said annular element (15).

4. Packaging and applicator unit according to Claim 1 or 2, **characterized in that** the thimble (11) and the annular element (15) are obtained by two-shot injection moulding of two physico-chemically compatible materials.

5. Packaging and applicator unit according to Claim 1, **characterized in that** the rigidifying means (20, 26-30) are secured to the annular element (15).

6. Packaging and applicator unit according to Claim 5, **characterized in that** the rigidifying means (20, 26-30) consist of an element situated facing all or part of the periphery of the thimble (11), and extending from the annular element (15) over part of the axial height of the applicator member (10).

7. Packaging and applicator unit according to Claim 6, **characterized in that** the rigidifying means consist of a cylindrical sleeve (20).

8. Packaging and applicator unit according to Claim 7, **characterized in that** the said sleeve (20) extends over approximately 1/4 to 1/3 of the axial height of the applicator member (10).

9. Packaging and applicator unit according to Claim 7 or 8, **characterized in that** the annular element (15), the rigidifying means (20, 26-30) and the thimble (11) form a single piece obtained by moulding of a thermoplastic material, the thimble (11) having a region of greater thickness extending from the annular element (15) in such a way as to form the rigidifying means (20), and a region of lesser thickness extending from the region of greater thickness as far as a closed end (12) formed by the said thimble (11).

10. Packaging and applicator unit according to Claim 5 or 6, **characterized in that** the rigidifying means consist of at least two tabs (29, 30) directed along the axis X.

11. Packaging and applicator unit according to Claim 10, **characterized in that** the said tabs (29, 30) extend over approximately 1/4 to 3/4 of the axial height of the applicator member (10).

12. Packaging and applicator unit according to any one of Claims 1 to 11, **characterized in that** the said rigidifying means (20, 26-30) are situated on the outside of the thimble (11).

13. Packaging and applicator unit according to any one of the preceding claims, **characterized in that** the means for, in a storage position, allowing the said annular element (15) to be positioned near the said opening (19) when the said applicator member is arranged inside.the container, comprise an annular groove (16) formed in a transverse flange (17) formed by a free edge (18) of a neck (4) surmounting the container and capable of accommodating the said annular element (15).

14. Packaging and applicator unit according to Claim 13, **characterized in that** the plugging means (8) comprise a stopper having an internal skirt (21) capable, when the plugging means (8) are in the closed position, of pressing on the said annular element (15) so as to hold it in the said groove (16).

15. Packaging and applicator unit according to Claim 14, **characterized in that** the stopper (8) is screwed onto the neck (4) of the container.

16. Packaging and applicator unit according to any one of the preceding claims, **characterized in that** the thimble (11) is made of a material such as low-density polyethylenes, latices, thermoplastic elastomers, nonwovens made of polyethylene or polypropylene, etc.

17. Packaging and applicator unit according to any one of the preceding claims, **characterized in that** the product is picked up near a closed end (12) of the thimble (11) which is the opposite end to the open end (14), the thimble being, at least near the said closed end (12), of a thickness of between 0.1 mm and 1 mm.

18. Use of a packaging and applicator unit according to any one of the preceding claims for a cosmetic product, particularly a care cream, a gel or a foundation.
